(19) Europäisches Patentamt
European Patent Office
Office européen des brevets

(11) EP 1 749 819 A1

(12) EUROPEAN PATENT APPLICATION

(43) Date of publication:
**07.02.2007 Bulletin 2007/06**

(21) Application number: **05384011.2**

(22) Date of filing: **15.07.2005**

(51) Int Cl.:
*C07D 231/06* (2006.01) *A61K 31/4155* (2006.01)
*A61P 3/04* (2006.01) *A61P 25/30* (2006.01)
*A61P 35/00* (2006.01)

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **LABORATORIOS DEL DR. ESTEVE, S.A.**
**08041 Barcelona (ES)**

(72) Inventors:
- **Torrens Jover, Antonio**
  **08221 Terrasa (Barcelona) (ES)**
- **Buschmann, Helmut Heinrich**
  **08960-Sant Just Desvern, Barcelona (ES)**
- **Sola, Lluis**
  **Av. Països Catalans 16, 43007 Tarragona (ES)**
- **Benet Buschholz, Jordi**
  **Av. Països Catalans 16, 43007 Tarragona (ES)**

# (54) (rac)-N-piperidinyl-5-(4-chlorophenyl)-1-(2,4-dichlorophenyl)-4,5-dihydro-1H-pyrazole-3-carboxamide hydrates

(57) The present invention relates to (rac)-N-piperidinyl-5-(4-chlorophenyl)-1-(2,4-dichlorophenyl)-4, 5-dihydro-1H-pyrazole-3-carboxamide • X $H_2O$, with X between 0.7 and 0.3.
Especially preferred is (rac)-N-piperidinyl-5-(4-chlorophenyl)-1-(2,4dichlorophenyl)-4, 5-dihydro-1H-pyrazole-3-carboxamide • monhydrate.
Methods of preparation and their use as modulators of the cannabinoid receptor are also disclosed.

**Figure 1**

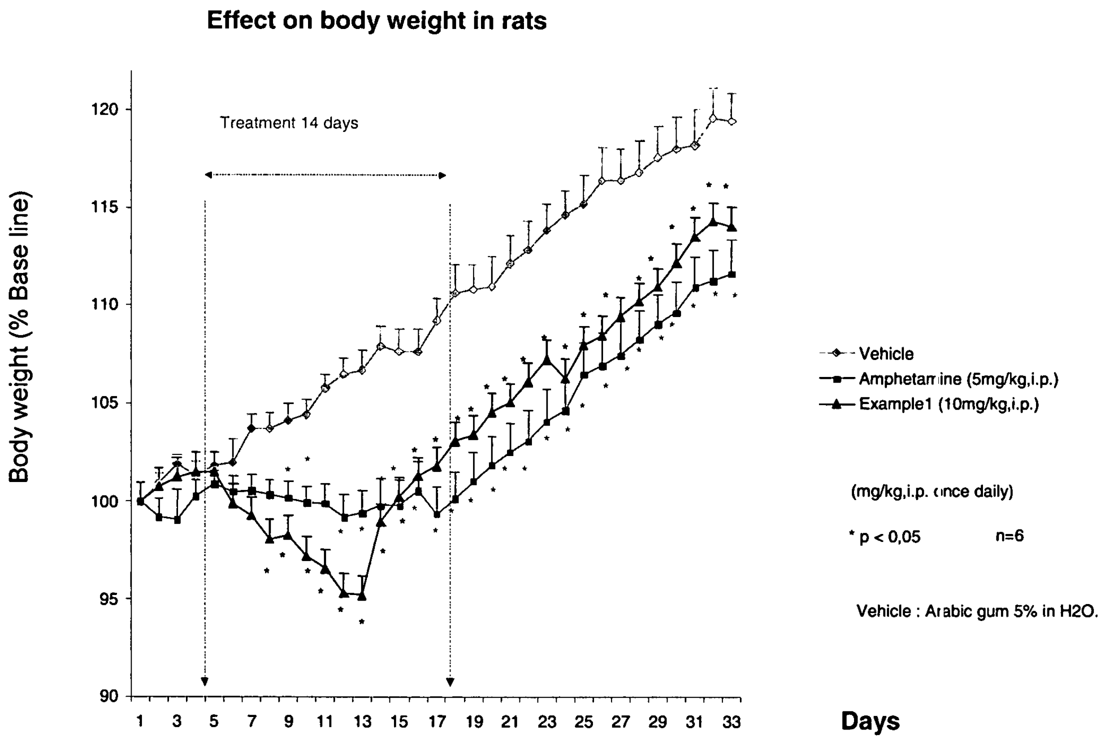


EP 1 749 819 A1

## Description

The present invention relates to substituted pyrazoline hydrates, methods for their preparation, medicaments comprising these compounds as well as their use for the preparation of a medicament for the treatment of humans and animals.

Cannabinoids are compounds, which are derived from the cannabis sativa plant which is commonly known as marijuana. The most active chemical compound of the naturally occurring cannabinoids is tetrahydrocannabinol (THC), particularly $\Delta^9$-THC.

These naturally occuring cannabinoids as well as their synthetic analogues promote their physiological effects via binding to specific G-coupled receptors, the so-called cannabinoid-receptors.

At present, two distinct types of receptors that bind both the naturally occurring and synthetic cannabinoids have been identified and cloned. These receptors, which are designated $CB_1$ and $CB_2$ are involved in a variety of physiological or pathophysiological processes in humans and animals, e.g. processes related to the central nervous system, immune system, cardiovascular system, endocrinous system, respiratory system, the gastrointestinal tract or to reproduction, as described for example, in Hollister, Pharm. Rev. 38, 1986, 1-20; Reny and Singha, Prog. Drug. Res., 36, 71-114, 1991; Consroe and Sandyk, in Marijuana/Cannabinoids, Neurobiology and Neurophysiology, 459, Murphy L. and Barthe A. Eds., CRC Press, 1992.

Therefore, compounds, which have a high binding affinity for these cannabinoid receptors and which are suitable for modulating these receptors are useful in the prevention and/or treatment of cannabinoid-receptor related disorders.

In particular, the $CB_1$-Receptor is involved in many different food-intake related disorders such as bulimia or obesity, including obesity associated with type II diabetes (non-insulin-dependent diabetes) and thus, compounds suitable for regulating this receptor may be used in the prophylaxis and/or treatment of these disorders.

Thus, it was an object of the present invention to provide novel compounds for use as active substances in medicaments. In particular, these active substances should be suitable for the modulation of Cannabinoid receptors, more particularly for the modulation of Cannabinoid 1 ($CB_1$) receptors.

Said object was achieved by providing the piperidinyl-pyrazoline hydrates given below.

It has been found that these compounds have a high affinity for cannabinoid receptors, particularly for the $CB_1$-receptor, and that they act as modulators e.g. antagonists, inverse agonists or agonists on these receptors. They are therefore suitable for the prophylaxis and/or treatment of various disorders related to the central nervous system, the immune system, the cardiovascular system, the endocrinous system, the respiratory system, the gastrointestinal tract or reproduction in humans and/or animals, preferably humans including infants, children and grown-ups.

The hydrates claimed are further especially stable.

Thus, in one of its aspects the present invention relates to (rac)-N-piperidinyl-5-(4-chlorophenyl)-1-(2,4-dichlorophenyl)-4,5-dihydro-1H-pyrazole-3-carboxamide • X $H_2O$, with X between 0.7 and 0.3.

Especially preferred is (rac)-N-piperidinyl-5-(4-chlorophenyl)-1-(2,4-dichlorophenyl)-4,5-dihydro-1H-pyrazole-3-carboxamide • monhydrate.

In another aspect the present invention also provides a process for the preparation of substituted pyrazoline hydrates, according to which at least one benzaldehyde compound of general formula II

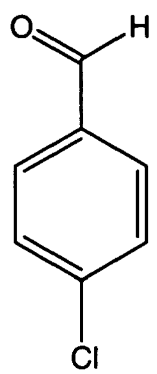

II

is reacted with a pyruvate compound of formula (III)

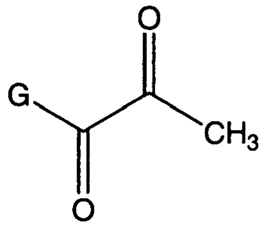

(III),

wherein G represents an OR group with R being a branched or unbranched $C_{1-6}$ alkyl radical or G represents an $O^-K$ group with K being a cation, to yield a compound of formula (IV)

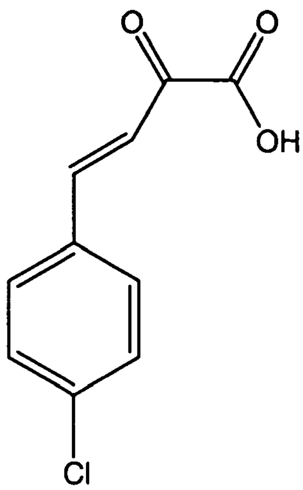

(IV)

which is optionally isolated and/or optionally purified, and which is reacted with an optionally substituted phenyl hydrazine of formula (V)

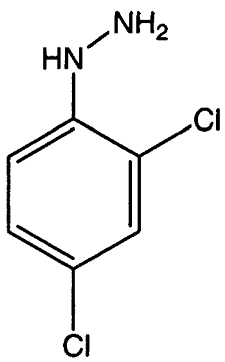

(V)

or a corresponding salt thereof, under inert atmosphere, to yield a compound of formula (VI)

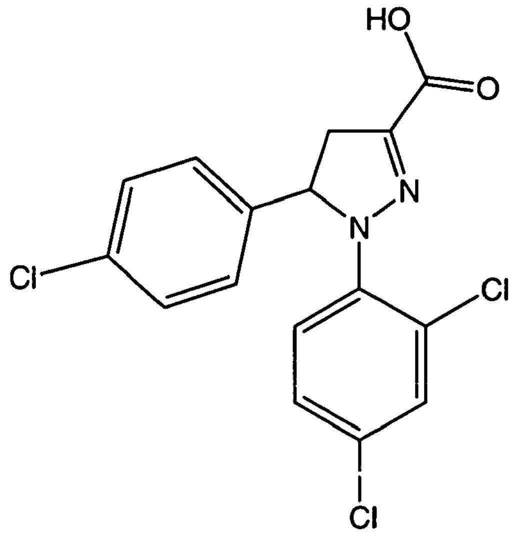

(VI)

which is optionally isolated and/or optionally purified, and optionally transferred under inert atmosphere to a compound of formula (VII) via the reaction with an activating agent

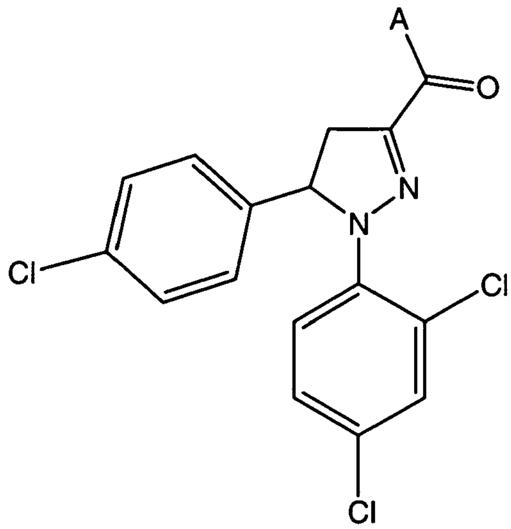

(VII)

wherein A represents a leaving group, said compound being optionally isolated and/or optionally purified, and at least one compound of formula (VII) is reacted with a compound of formula

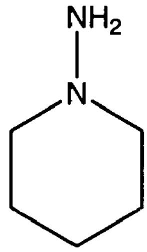

under inert atmosphere to yield (rac)-N-piperidinyl-5-(4-chlorophenyl)-1-(2,4-dichlorophenyl)-4,5-dihydro-1H-pyrazole-3-carboxamide, which is optionally isolated and/or optionally purified,
followed by an evaporation step in which (rac)-N-piperidinyl-5-(4-chlorophenyl)-1-(2,4-dichlorophenyl)-4,5-dihydro-1H-pyrazole-3-carboxamide is dissolved in an organic solvent which is slowly evaporated under temperatures below room temperature (25 ºC) to yield (rac)-N-piperidinyl-5-(4-chlorophenyl)-1-(2,4-dichlorophenyl)-4,5-dihydro-1H-pyrazole-3-carboxamide • X $H_2O$.

It is preferred if in the evaproration step the organic solvent is selected from:

- ethyl acetate,
- dichloromethane,
- 1,1,1-trichloroethane
- isobutyl acetate or
- ethyl acetate water.

It is preferred if in the evaproration step the temperature is lowered to between +5º and -20º C.

The reaction of the benzaldehyde compound of formula II with a pyruvate compound of formula III is preferably carried out in the presence of at least one base, more preferably in the presence of an alkali metal hydroxide such as sodium hydroxide or potassium hydroxide or an alkali metal methoxide such as sodium methoxide, as described, for example, in Synthetic communications, 26(11), 2229-33, (1996). The respective description is hereby incorporated by reference and forms part of the disclosure. Preferably sodium pyruvate may be used as the pyruvate compound. Preferably said reaction is carried out in a protic reaction medium such as a $C_{1-4}$ alkyl alcohol or mixtures of these. Mixtures of such alcohols with water, e.g. ethanol/water may also be used.

Reaction temperature as well as the duration of the reaction may vary over a broad range. Preferred reaction temperatures range from -10 °C to the boiling point of the reaction medium. Suitable reaction times may vary for example from several minutes to several hours.

Also preferred the reaction of the benzaldehyde compound of formula II with a pyruvate compound of formula III is carried out under acid catalysed conditions, more preferably by refluxing the mixture in dichloromethane in the presence of copper(II)trifluoromethanesulfonate as described, for example, in Synlett, (1), 147-149, 2001. The respective description is hereby incorporated by reference and forms part of the disclosure.

The reaction of the compound of formula (IV) with an substituted phenyl hydrazin of formula (V) is preferably carried out in a suitable reaction medium such as $C_{1-4}$-alcohols or ethers such as dioxane or tetrahydrofurane or mixtures of at least two of these afore mentioned compounds. Also preferably, said reaction may be carried out in the presence of an acid, whereby the acid may be organic such as acetic acid and/or inorganic such as hydrochloric acid. Furthermore, the reaction may also be carried out in the presence of a base such as piperidine, piperazine, sodium hydroxide, potassium hydroxide, sodium methoxide or sodium ethoxide, or a mixture of at least two of these bases may also be used.

Reaction temperature as well as the duration of the reaction may vary over a broad range. Suitable reaction temperatures range from room temperature, i.e. approximately 25 °C to the boiling point of the reaction medium. Suitable reaction times may vary for example from several minutes to several hours.

The carboxylic group of the compound of formula (VI) may be activated for further reactions by the introduction of a suitable leaving group according to conventional methods well known to those skilled in the art. Preferably the compounds of formula (VI) are transferred into an acid chloride, an acid anhydride, a mixed anhydride, a $C_{1-4}$ alkyl ester, an activated ester such as p-nitrophenylester. Other well known methods for the activation of acids include the activation with N,N-dicyclohexylcarbodiimide or benzotriazol-N-oxotris(dimethylamino) phosphonium hexafluorophosphate (BOP)).

If said activated compound of formula (VII) is an acid chloride, it is preferably prepared by reaction of the

corresponding acid of formula (VI) with thionyl chloride or oxalyl chloride, whereby said chlorinating agent is also used as the solvent. Also preferably an additional solvent may be used. Suitable solvents include hydrocarbons such as benzene, toluene or xylene, halogenated hydrocarbons such as dichloromethane, chloroform or carbon tetrachloride, ethers such as diethyl ether, dioxane, tetrahydrofurane or dimethoxyethane. Mixtures of two or more solvents from one class or two or more solvents from different classes may also be used. Preferred reaction temperature range from 0º C to the boiling point of the solvent and reaction times from several minutes to several hours.

If said activated compound of formula (VII) is a mixed anhydride, said anhydride may preferably be prepared, for example, by reaction of the corresponding acid of formula (VI) with ethyl chloroformiate in the presence of a base such as triethylamine or pyridine, in a suitable solvent.

The reaction of general formula (VII) with

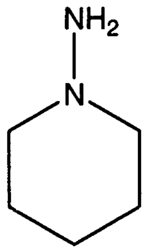

to yield compounds according to the invention is preferably carried out in presence of a base such as triethylamine in a reaction medium such as methylenchloride. The temperature is preferably in the range from 0ºC to the boiling point of the reaction medium. The reaction time may vary over a broad range, e.g. from several hours to several days.

The afore mentioned reactions involving the synthesis of the 4,5-dihydro-pyrazole ring or the reaction of a compound comprising said ring are carried out under an inert atmosphere, preferably nitrogen or argon, to avoid oxidation of the ring-system.

During the processes described above the protection of sensitive groups or of reagents may be necessary and/or desirable. The introduction of conventional protective groups as well as their removal may be performed by methods well-known to those skilled in the art.

Those skilled in the art understand that the term substituted pyrazoline hydrates as used herein is to be understood as encompassing derivatives such as ethers, esters and complexes of these compounds as well. The term "derivatives" as used in this application is defined here as meaning a chemical compound having undergone a chemical derivation starting from an acting (active) compound to change (ameliorate for pharmaceutical use) any of its physico-chemical properties, especially a so-called prodrug, e.g. their esters and ethers. Examples of well known methods of producing a prodrug of a given acting compound are known to those skilled in the art and can be found e.g. in Krogsgaard-Larsen et al., Textbook of Drugdesign and Discovery, Taylor & Francis (April 2002). The respective description is hereby incorporated by reference and forms part of the disclosure.

The purification and isolation of the inventive substituted pyrazoline hydrates or any intermediate thereof may, if required, be carried out by conventional methods known to those skilled in the art, e.g. chromatographic methods or recrystallization.

The substituted pyrazoline hydrates according to the invention, are suitable as pharmaceutical active substances for the preparation of medicaments.

It has been found that the substituted pyrazoline compounds (hydrates) have a high affinity to cannabinoid receptors, particularly cannabinoid 1 ($CB_1$)-receptors, i.e. they are selective ligands for the ($CB_1$)-receptor and act as modulators, e.g. antagonists, inverse agonists or agonists, on these receptors. In particular, these pyrazoline compounds show little or no development of tolerance during treatment, particularly with respect to food intake, i.e. if the treatment is interrupted for a given period of time and then continued afterwards, the inventively used pyrazoline compounds will again show the desired effect. After ending the treatment with the pyrazoline compounds, the positive influence on the body weight is found to continue.

Furthermore, these pyrazoline compounds show relatively weak Herg channel affinity, thus a low risk of prolongation of the QT-interval is to be expected for these compounds.

In summary, the inventively used substituted pyrazoline compounds (hydrates) are distinguished by a broad spectrum of beneficial effects, while at the same time showing relatively little undesired effects, i.e. effects which do not positively contribute to or even interfere with the well being of the patient.

Thus, another aspect of the present invention relates to a medicament comprising at least one substituted pyrazoline hydrate according to the invention and optionally one or more pharmaceutically acceptable excipients.

Another aspect of the present invention is the use of at least one of the substituted pyrazoline hydrates according to the invention, and optionally one or more pharmaceutically acceptable excipients, for the preparation of a medicament for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

Also preferred is the use of at least one of the substituted pyrazoline hydrates according to the invention, and optionally one or more pharmaceutically acceptable excipients, for the prophylaxis and/or treatment of psychosis.

Also particularly preferred is the use of at least one of the substituted pyrazoline hydrates according to the invention, and optionally one or more pharmaceutically acceptable excipients, for the preparation of a medicament for the prophylaxis and/or treatment of food intake disorders, preferably bulimia, anorexia, cachexia, obesity and/or type II diabetus mellitus (non-insuline dependent diabetes mellitus), more preferably obesity.

Also preferred is the use of at least one of the substituted pyrazoline hydrates according to the invention, and optionally one or more pharmaceutically acceptable excipients, for the preparation of a medicament for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

Also preferred is the use of at least one of the substituted pyrazoline hydrates according to the invention, and optionally one or more pharmaceutically acceptable excipients, for the preparation of a medicament for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

Medicaments/drugs, which are frequently the subject of misuse include opioids, barbiturates, cannabis, cocaine, amphetamines, phencyclidine, hallucinogens and benzodiazepines.

Also preferred is the use of at least one of the substituted pyrazoline hydrates according to the invention, and optionally one or more pharmaceutically acceptable excipients, for the preparation of a medicament for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, , medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

Also preferred is the use of at least one of the substituted pyrazoline hydrates according to the invention, and optionally one or more pharmaceutically acceptable excipients, for the preparation of a medicament for the prophylaxis and/or treatment of metabolic syndrome, especially weight independent, cardiovascular diseases, cardiovascular risk factors, glucose intolerance and insulin resistance; for the prophylaxis and/or treatment and/or influencing of blood parameters especially lipid parameters, seeming to lower LDL bodies while increasing HDL bodies, including also the treatment of food dispredrs (obesity) under conditions of developed diabetes, especially Type II.

Another preferred aspect of the invention is also a method of treatment encompassing all the abovementioned uses, wherein the substituted pyrazoline hydrates according to the invention is applied to a person in need thereof, treating metabolic syndrome, especially weight independent, cardiovascular diseases especially fighting cardiovascular risk factors, influencing the blood parameters, especially the lipid parameters, diabetes, especially type II, glucose intolerance and insulin resistance, bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit; alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction; cancer, preferably

for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer; food intake disorders, preferably bulimia, anorexia, cachexia, obesity and/or type II diabetus mellitus (non-insuline dependent diabetes mellitus), more preferably obesity; psychosis; disorders of the central nervous system, disorders of the immune system, disorders of the cardiovascular system, disorders of the endocrinous system, disorders of the respiratory system, disorders of the gastrointestinal tract or reproductive disorders.

The medicament according to the present invention may be in any form suitable for the application to humans and/or animals, preferably humans including infants, children and adults and can be produced by standard procedures known to those skilled in the art. The composition of the medicament may vary depending on the route of administration.

The medicament of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions, emulsions, or dry powdered forms suitable for reconstitution with water or other suitable liquid medium before use, for immediate or retarded release.

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000 milligrams of active substance to be administered during one or several intakes per day.

**Pharmacological Methods**

**I. In-vitro determination of affinity to CB1/CB2-Receptors**

The in-vitro determination of the affinity of the inventive substituted pyrazoline compounds to $CB_1$/$CB_2$-Rezeptors is carried out as described in the publication of Ruth A. Ross, Heather C. Brockie et al., "Agonist-inverse agonist characterization at CB1 and CB2 cannabinoid receptors of L-759633, L759656 and AM630", British Journal of Pharmacology, 126, 665-672, (1999), whereby the transfected human $CB_1$ and $CB_2$ receptors of Receptor Biology. Inc. are used. The radioligand used for both receptors is [$^3$H]-CP55940. The respective parts of the description is hereby incorporated by reference and forms part of the present disclosure.

**II. In-vivo bioassay system for determination of cannabinoid activity**

**Mouse tetrad model**

Substances with affinity for cannabinoid receptors are known to produce a wide range of pharmacological effects. It is also known that intravenous administration of a substance with affinity for cannabinoid receptors in mice produces analgesia , hypothermia, sedation and catalepsy. Individually, none of these effects can be considered as proof that a tested substance has affinity for cannabinoid-receptors, since all of these effects are common for various classes of centrally active aaents. However, substances, which show all of these effects, i.e. substances that are active in this so-called tetrad model are considered to have affinity for the cannabinoid receptors. It has further been shown that cannabinoid receptor antagonists are higly effective in blocking the effects of a cannabinoid agonist in the mouse tetrad model.

The tetrad model is described, for example, in the publication of A. C. Howlett et al, International Union of Pharmacology XXVII. Classification of Cannabinoid Receptors, Pharmacol Rev 54, 161-202 , 2002 and David R. Compton et al., "In-vivo Characterization of a Specific Cannabinoid Receptor Antagonist (SR141716A) :Inhibition of Tetrahydrocannbinol- induced Responses and Apparent Agonist Activity", J. Pharmacol. Exp. Ther. 277 , 2, 586-594, 1996. The corresponding parts of the description are hereby incorporated by reference.

**Material and Methods**

Male NMRI mice with a weight of 20-30 g (Harlan, Barcelona, Spain) are used in all of the following experiments.
Before testing in the behavioral procedures given below, mice are acclimatized to the experimental setting. Pre-Treatment control values are determined for analgesia hot plate latency (in seconds), rectal temperature, sedation and catalepsy.
In order to determine the agonistic activty of the substance to be tested, the mice are injected intravenously with the substance to be tested or the vehicle alone. 15 minutes after injection, latency in hot plate analgesia is measured. Rectal temperature, sedation and catalepsy are measured 20 minutes after injection.
In order to determine the antagonistic activity the identical procedure is used as for the determination of the agonistic effects, but with the difference that the substance to be evaluated for its antagonistic activity is injectected 5 minutes before the intravenous injection of 1.25 mg/kg Win-55,212 a known cannabinoid-receptor agonist.

**Hot plate analgesia**

The hot plate analgesia is determined according to the method described in Woolfe D. et al. "The evaluation of analgesic action of pethidine hydrochloride (Demerol)", J. Pharmacol. Exp. Ther. 80, 300-307,1944. The respective description is hereby incorporated by reference and forms part of the present disclosure.
The mice are placed on a hot plate (Harvard Analgesimeter) at 55 ± 0.5 °C until they show a painful sensation by licking their paws or jumping and the time for these sensations to occur is recorded. This reading is considered the basal value (B). The maximum time limit the mice are allowed to remain on the hot plate in absence of any painful response is 40 seconds in order to prevent skin damage. This period is called the cut-off time (PC).
Fifteen minuts after the administration of the substance to be tested, the mice are again placed on the hot plate and the afore described procedure is repeated. This period is called the post-treatment reading (PT).
The degree of analgesia is calculated from the formula:

$$\%\ \text{MPE of Analgesia} = (\text{PT} - \text{B}) / (\text{PC} - \text{B}) \times 100$$

MPE = Maximum possible effect.

**Determination of sedation and ataxia**

Sedation and ataxia is determined according to the method described in Desmet L. K. C. et al. "Anticonvulsive properties of Cinarizine and Flunarizine in Rats and Mice", Arzneim. -Forsch. (Frug Res) 25, 9, 1975. The respective description is hereby incorporated by reference and forms part of the present disclosure.
The chosen scoring system is

**0:** no ataxia;
**1:** doubful;
**2:** obvious calmness and quiet;
**3** pronounced ataxia;

prior to as well as after treatment.
The percentage of sedation is determined according to the formula:

$$\%\ \text{of sedation} = \text{arithmetic mean} / 3 \times 100$$

**Hypothermia:**

Hypothermia is determined according to the method described in David R. Compton et al. "In-vivo Characterization of a Specific Cannabinoid Receptor Antagonist (SR141716A) Inhibition of Tetrahydrocannbinol- induced Responses and Apparent Agonist Activity", J. Pharmacol Exp Ther. 277 , 2, 586-594, 1996. The respective description is hereby incorporated by reference and forms part of the present disclosure.

The base-line rectal temperatures are determined with a thermometer (Yello Springs Instruments Co., Panlabs) and a thermistor probe inserted to 25mm before the administration of the substance to be tested. Rectal temperature is again measured 20 minutes after the administration of the substances to be tested. The temperature difference is calculated for each animal, whereby differences of ≥ -2 ºC are considered to represent activity.

**Catalepsy:**

Catalepsy is determined according to the method described in Alpermann H. G. et al. "Pharmacological effets of Hoe 249: A new potential antidepressant", Drugs Dev. Res. 25, 267-282. 1992. The respective description is hereby incorporated by reference and forms part of the present disclosure.

The cataleptic effect of the substance to be tested is evaluated according to the duration of catalepsy, whereby the animals are placed head downwards with their kinlegs upon the top of the wooden block.

The chosen scoring system is:

Catalepsy for:
more than 60 seconds = 6; 50 -60 seconds = 5, 40-50 seconds = 4, 30-40 seconds = 3, 20-30 seconds = 2, 5-10 seconds = 1, and less than 5 seconds =0.

The percentage of catalepsy is determined according ot the following formula:

$$\% \text{ Catalepsy} = \text{arithmetic mean} / 6 \times 100$$

**III. In vivo testing for antiobesic activity**

The in-vivo testing for antiobesic activity of the inventive pyrazoline compounds is carried out as described in the publication of G. Colombo et al., "Appetite Suppression and Weight Loss after the Cannabinoid Antagonist SR 141716"; Life Sciences, 63 (8), 113-117, (1998). The respective part of the description is hereby incorporated by reference and forms part of the present disclosure.

**IV. In vivo testing for antidepressant activity**

The in-vivo testing for antidepressant activity of the inventive pyrazoline compounds in the water despair test is carried out as described in the publication of E.T. Tzavara et al., "The CB1 receptor antagonist SR141716A selectively increases monoaminergic neurotransmission in the medial prefrontal cortex: implications for therapeutic actions"; Br. J. Pharmacol. 2003, 138(4):544:53. The respective part of the description is hereby incorporated by reference and forms part of the present disclosure.

The present invention is illustrated below with the aid of examples. These illustrations are given solely by way of example and do not limit the general spirit of the present invention.

**Examples:**

**Example 1:**

**N-piperidinyl-5-(4-chlorophenyl)-1-(2,4-dichlorophenyl)-4,5-dihydro-1H-pyrazole-3-carboxamide**

**a) 4-(4-chlorophenyl)-2-oxo-3-butenoic acid**

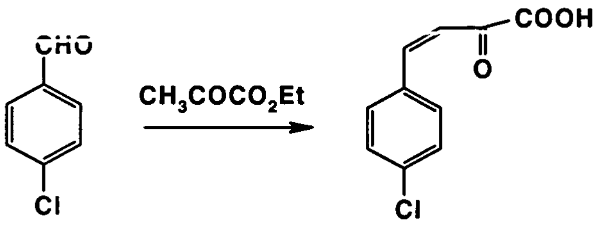


In a three neck flask p-chlorobenzaldehyde (13,3 g, 95 mmoles) and ethyl pyruvate (10 g, 86 mmoles) were dissolved in 150 ml of absolute ethanol.The solution was ice-cooled to 0°C and an aqueous solution of NaOH (3.8 g in 45 mL water) was added dropwise keeping the temperature below or equal to 10°C, whereby a yellow-orange colored precipitate was formed. The reaction mixture was stirred for 1 hour at 0°C and an additional 1.5 hours at room temperature (approximately 25 °C). Afterwards the reaction mixture was cooled down to approximately 5°C and the insoluble sodium salt of 4-(4-chlorophenyl)-2-oxo-3-butenoic acid was isolated by filtration.

The filtrate was left in the refrigerator overnight, whereby more precipitate is formed, which was filtered off, combined with the first fraction of the salt and washed with diethyl ether. The sodium salt of 4-(4-chlorophenyl)-2-oxo-3-butenoic acid was then treated with a solution of 2N HCl, stirred for some minutes and solid 4-(4-chlorophenyl)-2-oxo-3-butenoic acid was separated via filtration and dried to give 12.7 g of the desired product (70% of theoretical yield).

IR (KBr, $cm^{-1}$): 3500-2500, 1719,3, 1686,5, 1603,4, 1587,8, 1081,9.
$^1H$ NMR($CDCl_3$, δ): 7,4 (d, J=8,4Hz, 2H), 7,5 (d, J=16,1Hz, 1 H), 7,6 (d, J=8,4Hz, 2H), 8,1 (d, J=16,1Hz, 1H).

**b) 5-(4-chlorophenyl)-1-(2,4-dichlorophenyl)-4,5-dihydro-pyrazole-3-carboxylic acid**

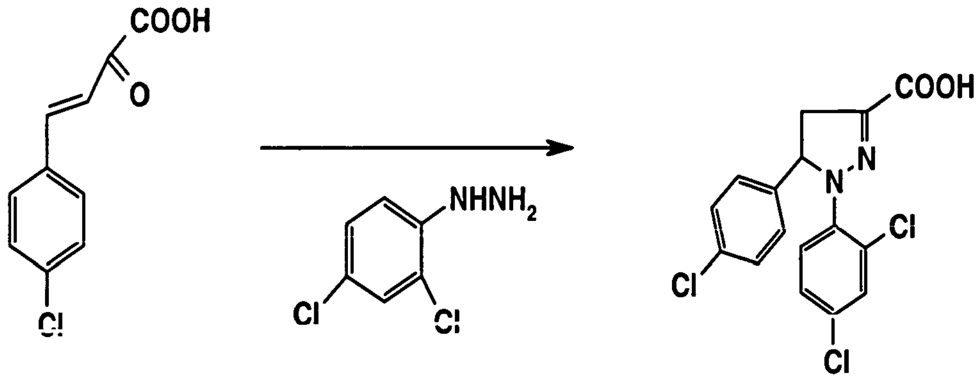

4-(4-chlorophenyl)-2-oxo-3-butenoic acid obtained according to step a) (12.6 g, 60 mmoles), 2,4-dichlorophenylhydrazine hydrochloride (12.8 g, 60 mmoles) and glacial acetic acid (200 mL) were mixed under a nitrogen atmosphere and heated to reflux for 4 hours, cooled down to room temperature (approximately 25 °C) and given into ice-water, whereby a sticky mass was obtained, which was extracted with methylene chloride. The combined methylene chloride fractions were washed with water, dried with sodium sulfate, filtered and evaporated to dryness to give a pale yellow solid (12.7 g, 57% of theoretical yield).

IR (KBr, $cm^{-1}$): 3200-2200, 1668,4, 1458, 1251,4, 1104,8.
$^1H$ NMR ($CDCl_3$, δ): 3,3 (dd, 1H), 3,7 (dd, 1 H), 5,9 (dd, 1 H), 7,09-7,25 (m, 7H).

**(c) 5-(4-chlorophenyl)-1-(2,4-dichlorophenyl)-4,5-dihydro-pyrazole-3-carboxylic acid chloride**

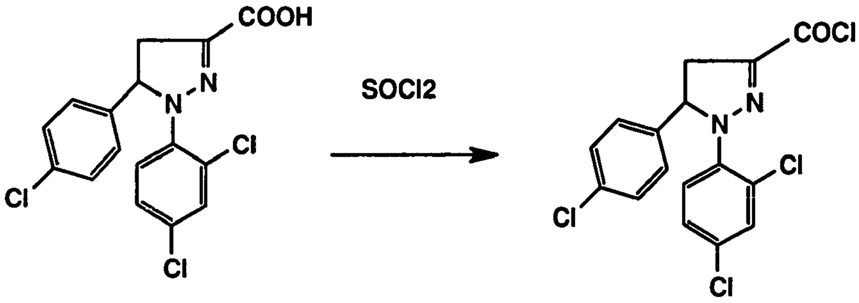


Under nitrogen atmosphere 5-(4-chlorophenyl)-1-(2,4-dichlorophenyl)-4,5-dihydro-pyrazole-3-carboxylic acid (2.5 g, 6.8 mmols) obtained according to step (b) was dissolved in 4 mL of in thionyl chloride and heated to reflux for 2.5 hours. The excess thionyl chloride is removed from the reaction mixture under reduced pressure and the resulting crude residue (2.6 g) is used without any further purification.
IR (KBr, $cm^{-1}$): 1732,3, 1700, 1533,3, 1478,1, 1212,9, 826,6.

**d) N-piperidinyl-5-(4-chlorophenyl)-1-(2,4-dichlorophenyl)-4,5-dihydropyrazole-3-carboxamide** [this compound may also be referred to as 5-(4-Chloro-phenyl)-1-(2,4-dichloro-phenyl)-4,5-dihydro-1H-pyrazole-3-carboxylic acid piperidin-1-ylamide or as 1-(2,4-dichlorophenyl)-5-(4-chlorophenyl)-4,5-dihydro-N-(piperidin-1-yl)-1H-pyrazole-3-carboxamide]

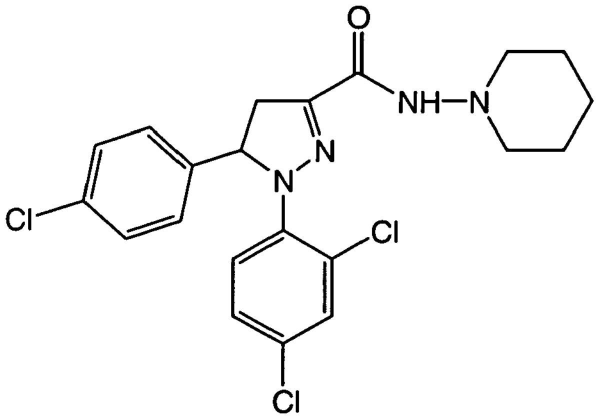

Under nitrogen atmosphere N-aminopiperidine (0.6 mL, 5.6 mmoles) and triethylamine (4 mL) were dissolved in methylene chloride (25 mL). The resulting mixture was ice-cooled down to 0°C and a solution of 5-(4-chlorophenyl)-1-(2,4-dichlorophenyl)-4,5-dihydro-pyrazole-3-carboxylic acid chloride obtained in step (c) in methylene chloride (15 mL) was added dropwise. The resulting reaction mixture was stirred at room temperature (approximately 25 °C) overnight. Afterwards the reaction mixture was washed with water, followed by a saturated aqueous solution of sodium bicarbonate, then again with water, dried over sodium sulfate, filtered and evaporated to dryness in a rotavapor. The resulting crude solid was crystallized from ethanol. The crystallized solid was removed via filtration and the mother liquors were concentrated to yield a second fraction of crystallized product. The two fractions were combined to give a total amount of 1.7 g (57% of theoretical yield) of N-piperidinyl-5-(4-chlorophenyl)-1-(2,4-dichlorophenyl)-4,5-dihydropyrazole-3-carboxamide having a melting point of 183-186°C.
IR (KBr, $cm^{-1}$): 3222,9, 2934,9, 1647,4, 1474,7, 1268,3, 815,6.
$^1H$ NMR ($CDCl_3$, δ): 1,4 (m, 2H), 1,7 (m, 4H), 2,8 (m, 4H), 3,3 (dd, J=6,1 y 18,3Hz, 1 H), 3,7 (dd, J=12,5 and 18,3 Hz, 1 H), 5,7 (dd, J=6.1 and 12,5 Hz, 1H), 7,0-7,2 (m, 6H), 7,4 (s, 1H).

**Example 2:**

**N-piperidinyl-5-(4-chlorophenyl)-1-(2,4-dichlorophenyl)-4,5-dihydro-1H-pyrazole-3-carboxamide • monohydrate**

a) The pure monohydrate was obtained by slow evaporation of a solution of the compound according to example 1 (N-piperidinyl-5-(4-chlorophenyl)-1-(2,4-dichlorophenyl)-4,5-dihydro-1H-pyrazole-3-carboxamide) in:

aa) dichlormethane,
bb) 1,1,1-trichloroethane and
cc) isobutylactetate

at - 18ºC.
b) Additionally monohydrate with impurities was obtained by slow evaporation of ethyl acetate.
c) Pure monohydrate can be generated in a reproducible way from a solution of the compound according to example 1 (N-piperidinyl-5-(4-chlorophenyl)-1-(2,4-dichlorophenyl)-4,5-dihydro-1H-pyrazole-3-carboxamide) in ethyl acetate water at 3ºC.

**Example 3:**

**Crystal structure of N-piperidinyl-5-(4-chlorophenyl)-1-(2,4-dichlorophenyl)-4,5-dihydro-1H-pyrazole-3-carboxamide • monohydrate**

The monohydrate forms characteristic flat and, at the polarized light, coloured needles.
A summary of the cell constants is given in table 1 below:

| | Unit cell dimensions | |
|---|---|---|
| Triclinic P1 | a = 10.9653 (13) Å | α = 92.312(2)º. |
| Volume: 2158.5 (4) Å$^3$ | b = 11.2034 (13) Å | β = 96.067(2)º |
| Density: 1.446 Mg/m$^3$ | C = 17.705 (2) Å | γ = 92.545(3)º |

**Example 4:**

**DSC analysis of N-piperidinyl-5-(4-chlorophenyl)-1-(2,4-dichlorophenyl)-4,5-dihydro-1H-pyrazole-3-carboxamide • monohydrate**

The DSC analysis of the hydrate presents a broad peak between 114 ºC and 128 ºC and a melting peak with an onset of 184 ºC and a maximum at 185 ºC. The first peak corresponds to water loss, and is observed even in a sealed crucible.
The hydrate can thus be identified by this broad peak due to water loss.

**Example 5:**

**TG analysis of N-piperidinyl-5-(4-chlorophenyl)-1-(2,4-dichlorophenyl)-4,5-dihydro-1H-pyrazole-3-carboxamide • monohydrate**

The TG analysis of the hydrate presents a characteristic weight loss of 3.7% between 61 ºC and 108 ºC, which corresponds to the water weight of a monohydrate (theoretical water loss 3.8%).

**Example 6:**

**FTIR Spectrum of N-piperidinyl-5-(4-chlorophenyl)-1-(2,4-dichlorophenyl)-4,5-dihydro-1H-pyrazole-3-carboxamide • monohydrate**

The FTIR Spektrum of the hydrate shows characteristic bands at 35056, 3450, 1605 and 1536 $cm^{-1}$.

**Example 7:**

**FTRaman spectrum of N-piperidinyl-5-(4-chlorophenyl)-1-(2,4-dichlorophenyl)-4,5-dihydro-1H-pyrazole-3-carboxamide • monohydrate**

The FTRaman spectrum of the hydrate shows intense bands at 1647, 1588, 1396, 1271, 1252, 1114, 1097, 787 and 664 $cm^{-1}$ and characteristic Raman shift regions at around 1540 and 38 $cm^{-1}$.

**Pharmacological Data:**

**I. In-vitro determination of affinity to $CB_1/CB_2$-Rezeptors**

The affinity of the inventive substituted pyrazoline compounds to $CB_1/CB_2$ receptors was determined as described above. Some of the values obtained are given in the following table I:

**Table I:**

| Compound according to Example | $CB_1$-Receptor Radiologand:[$^3$H]-CP55940 | | $CB_2$-Receptor Radiologand:[$^3$H]-CP55940 | |
|---|---|---|---|---|
| | % Inhibition $10^{-6}$ M | $K_i$(nM) | % Inhibition $10^{-6}$ M | $K_i$(nM) |
| 1 | 93 % | < 25 | 33 % | > 1000 |

As can be seen from the values given in table 1 the inventive pyrazoline compounds are particularly suitable for regulating the $CB_1$-Receptor.

**II. In-vivo bioassay system for determination of cannabinoid activity**

The determinination of cannabinoid activity in-vivo was determined as described above. Some of the values obtained are given in the following table II:

**Table II:**

| Compound according to example: | dosis administered: 5 mg/kg i.v. Agonistic effect | | | | dosis administered 5 mg/kg i.v. prior to Win 55212-2 in a dose of 1,25mg/kg i.v. Antagonistic Effect | | | |
|---|---|---|---|---|---|---|---|---|
| | A | B | C. | D | A | B. | C | D |
| 1 | 0 | 0 | 0 | 0 | 74 | 100 | 100 | 100 |

i.v. intravenous

A: Hot-Plate test
B: Hypothermia
C: Catalepsy
D: Sedation

As can be seen from the values given in table II the inventive pyrazoline compounds show an antagonistic effect.

**III. In-vivo testing for antiobesic activity**

The in-vivo testing for antiobesic activity was carried out as described above, whereby four different groups of 10 rats each were treated as follows:

**Group I:**

Group was treated with vehicle, namely arabic gum (5 wt.-%) in water.

**Group II:**

The second group of rats was treated with the inventive compound N-piperidinyl-5-(4-chlorophenyl)-1-(2,4-dichlorophenyl)-4,5-dihydropyrazole-3-carboxamide according to Example 1. Said compound was administered intraperitoneally to the rats over a period of 14 days in a daily dosis of (10 mg/kg body weight).

**Group III:**

The third group of rats was treated with Amphetamine, an active ingredient known to reduce appetite. Said compound was administered intraperitoneally to the rats over a period of 14 days in a daily dosis of (5 mg/kg body weight).
As can be seen from **Figure 1** the body weight is lowered due to the administration of the inventive compound according to example 1 and this effect is also observed after the treatment is ended.
**Figure 2** shows the reduction of food intake due to the administration of the inventive compound according to example 1.

**IV. In vivo testing for antidepressant activity**

The in-vivo testing for antidepressant activity of the inventive pyrazoline compounds in the water despair test was carried out as described above. In particular, the compound according to example 1 displayed positive effects with respect to immobility time and struggling time.

**Claims**

1. (rac)-N-piperidinyl-5-(4-chlorophenyl)-1-(2,4-dichlorophenyl)-4,5-dihydro-1H-pyrazole-3-carboxamide • X $H_2O$, with X between 0.7 and 0.3.

2. (rac)-N-piperidinyl-5-(4-chlorophenyl)-1-(2,4-dichlorophenyl)-4,5-dihydro-1H-pyrazole-3-carboxamide • monhydrate.

3. Process for the manufacture of the substituted pyrazoline hydrates according to claims 1 or 2, **characterized in that** a benzaldehyde of formula II

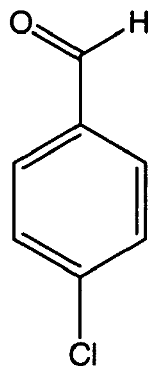

is reacted with a pyruvate compound of formula (III)

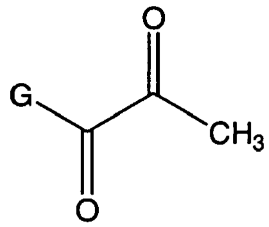

(III),

wherein G represents an OR group with R being a branched or unbranched $C_{1-6}$ alkyl radical or G represents an $O^-K$ group with K being a cation, to yield a compound of formula (IV)

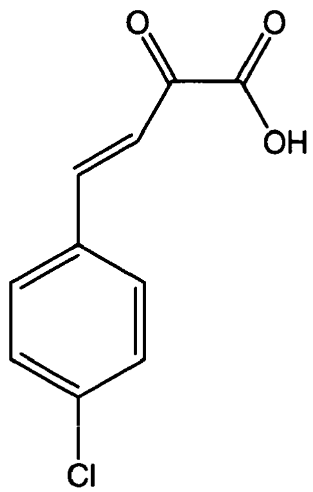

(IV)

which is optionally isolated and/or optionally purified, and which is reacted with an optionally substituted phenyl hydrazine of formula (V)

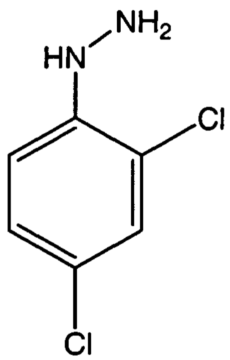

(V)

or a corresponding salt thereof, under inert atmosphere, to yield a compound of formula (VI)

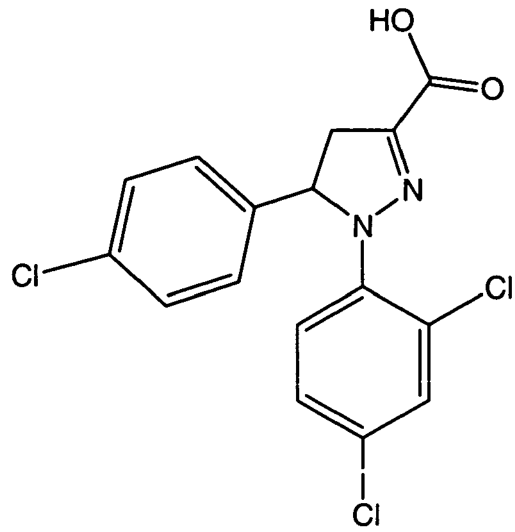

(VI)

which is optionally isolated and/or optionally purified, and optionally transferred under inert atmosphere to a compound of formula (VII) via the reaction with an activating agent

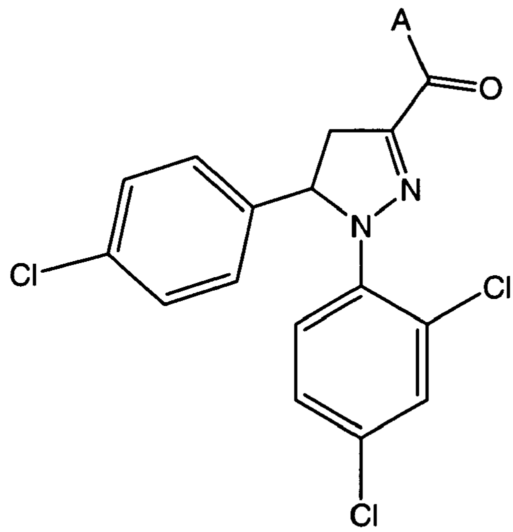

(VII)

wherein A represents a leaving group, said compound being optionally isolated and/or optionally purified, and at least one compound of formula (VII) is reacted with a compound of formula

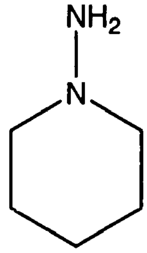

under inert atmosphere to yield (rac)-N-piperidinyl-5-(4-chlorophenyl)-1-(2,4-dichlorophenyl)-4,5-dihydro-1H-pyrazole-3-carboxamide, which is optionally isolated and/or optionally purified,
followed by an evaporation step in which (rac)-N-piperidinyl-5-(4-chlorophenyl)-1-(2,4-dichlorophenyl)-4,5-dihydro-1H-pyrazole-3-carboxamide is dissolved in an organic solvent which is slowly evaporated under temperatures below room temperature (25 ºC) to yield (rac)-N-piperidinyl-5-(4-chlorophenyl)-1-(2,4-dichlorophenyl)-4,5-dihydro-1H-pyrazole-3-carboxamide • X $H_2O$.

**4.** Process according to claim 3, **characterized in that** in the resolution step the organic solvent is selected from:

- ethyl acetate,
- dichloromethane,
- 1,1,1-trichloroethane
- isobutyl acetate or
- ethyl acetate water.

**5.** Process according to any of claims 3 or 4, **characterized in that** in the resolution step the temperature is lowered to between +5º and -20ºC.

**6.** Medicament comprising at least one substituted pyrazoline hydrate according to claim 1 or 2 and optionally of general formula I, and optionally one or more pharmaceutically acceptable excipients.

**7.** Use of the substituted pyrazoline hydrate according to one of claims 1 or 2 for the preparation of a medicament for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

**8.** Use of the substituted pyrazoline hydrate according to one of claims 1 or 2 for the preparation of a medicament for the prophylaxis and/or treatment of food intake disorders, preferably bulimia, anorexia, cachexia, obesity, type II diabetus mellitus (non-insuline dependent diabetes mellitus), more preferably obesity.

**9.** Use of the substituted pyrazoline hydrate according to one of claims 1 or 2, for the preparation of a medicament for the prophylaxis and/or treatment of psychosis.

**10.** Use of the substituted pyrazoline hydrate according to one of claims 1 or 2 for the preparation of a medicament for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

**11.** Use of the substituted pyrazoline hydrate according to one of claims 1 or 2, for the preparation of a medicament for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

**12.** Use of the substituted pyrazoline hydrate according to one of claims 1 or 2, for the preparation of a medicament for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

Figure 1

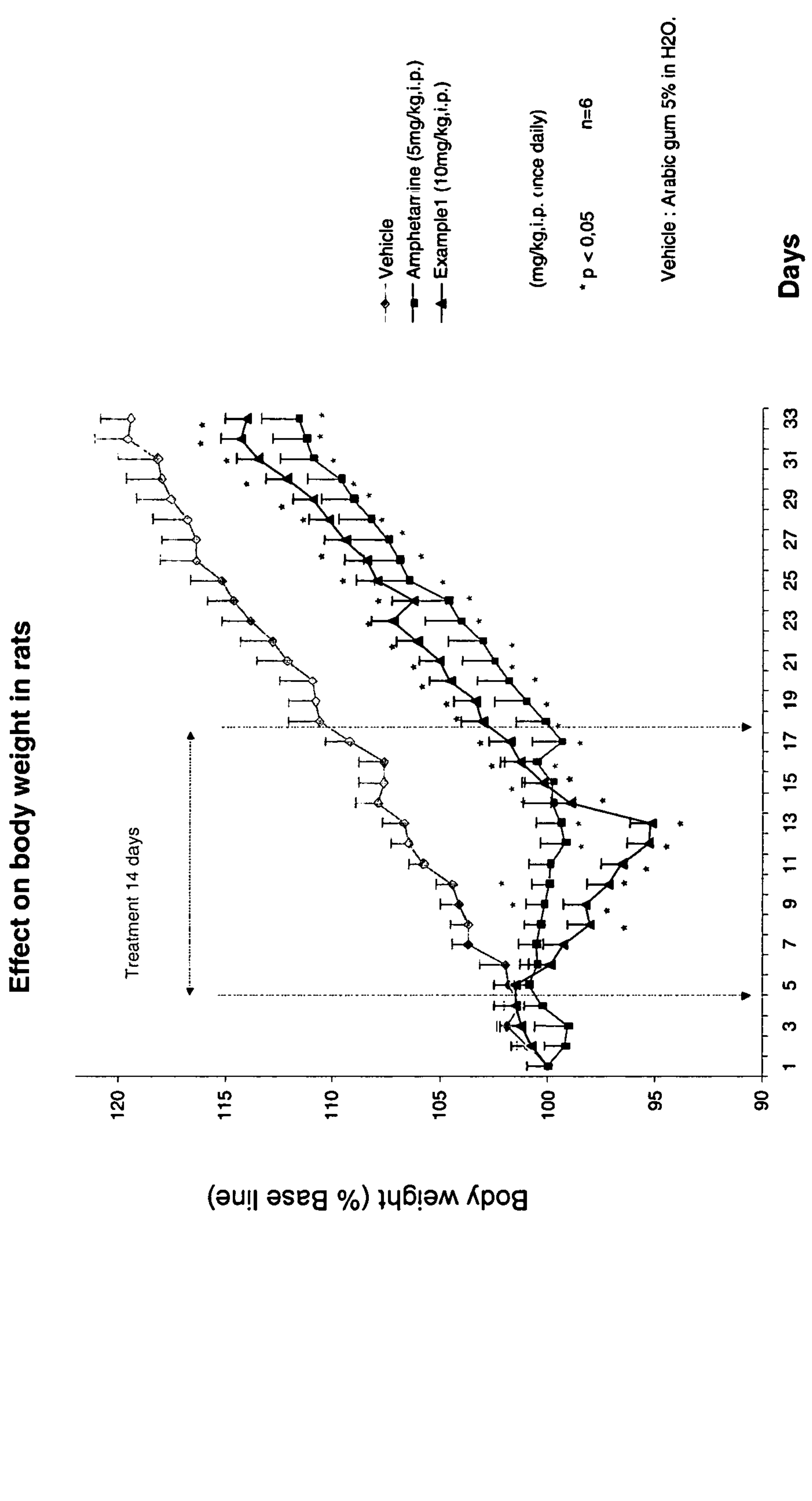
Effect on body weight in rats
Treatment 14 days
Body weight (% Base line)
120
115
110
105
100
95
90
1 3 5 7 9 11 13 15 17 19 21 23 25 27 29 31 33
Days
Vehicle
Amphetamine (5mg/kg,i.p.)
Example1 (10mg/kg,i.p.)
(mg/kg,i.p. once daily)
* p < 0,05 n=6
Vehicle : Arabic gum 5% in H2O.

Figure 2:

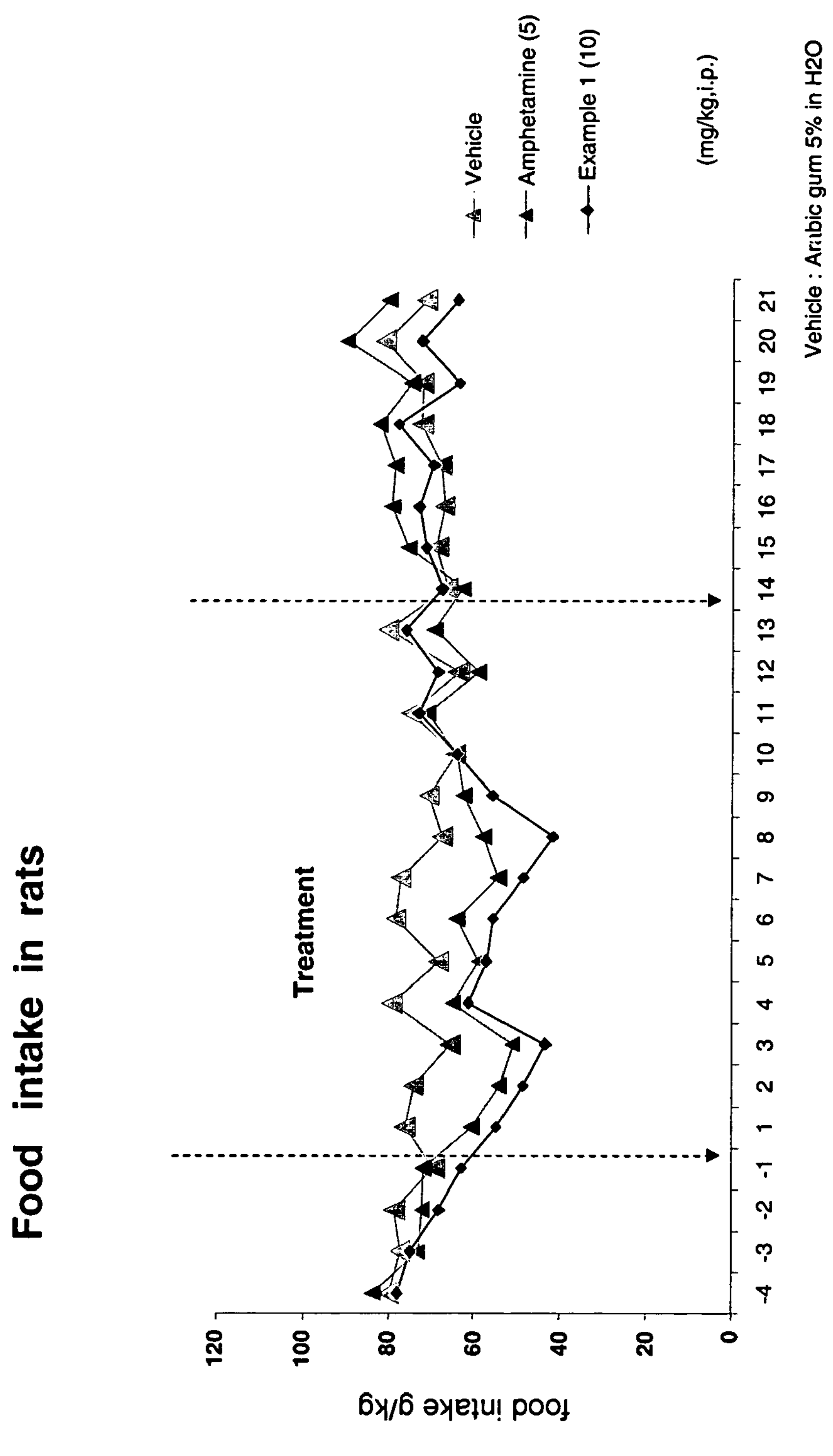
Food intake in rats
Treatment
food intake g/kg
0
20
40
60
80
100
120
-4 -3 -2 -1 1 2 3 4 5 6 7 8 9 10 11 12 13 14 15 16 17 18 19 20 21
Days
Vehicle
Amphetamine (5)
Example 1 (10)
(mg/kg,i.p.)
Vehicle : Arabic gum 5% in H2O

European Patent Office

# EUROPEAN SEARCH REPORT

Application Number
EP 05 38 4011

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| E | WO 2005/077911 A (LABORATORIOS DEL DR. ESTEVE S.A; CUBERES ALTISEN, ROSA; FRIGOLA CONSTA) 25 August 2005 (2005-08-25)<br>see example 1, data and figures and page 25, second paragraph and claim 9<br>----- | 1-12 | C07D231/06<br>A61K31/4155<br>A61P3/04<br>A61P25/30<br>A61P35/00 |
| A | WO 03/026647 A (SOLVAY PHARMACEUTICALS B.V; KRUSE, CORNELIS, G; LANGE, JOSEPHUS, H.M;) 3 April 2003 (2003-04-03)<br>* the whole document *<br>----- | 1-12 | |
| E | WO 2005/074920 A (SOLVAY PHARMACEUTICALS B.V; LANGE, JOSEPHUS H.M; KRUSE, CORNELIS G; VA) 18 August 2005 (2005-08-18)<br>see general formula and compound 2<br>----- | 1-12 | |
| X | PERTWEE R G: "PHARMACOLOGY OF CANNABINOID RECEPTOR LIGANDS"<br>CURRENT MEDICINAL CHEMISTRY, BENTHAM SCIENCE PUBLISHERS BV, BE,<br>vol. 6, no. 8, 1999, pages 635-664, XP009024021<br>ISSN: 0929-8673<br>see especially page 641<br>----- | 1-12 | TECHNICAL FIELDS SEARCHED (IPC)<br>C07D<br>A61K |

The present search report has been drawn up for all claims

1

| Place of search | Date of completion of the search | Examiner |
|---|---|---|
| Munich | 29 November 2005 | Scruton-Evans, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 05 38 4011

This annex lists the patent family members relating to the patent documents cited in the above-mentioned European search report.
The members are as contained in the European Patent Office EDP file on
The European Patent Office is in no way liable for these particulars which are merely given for the purpose of information.

29-11-2005

| Patent document cited in search report | Publication date | Patent family member(s) | Publication date |
|---|---|---|---|
| WO 2005077911 A | 25-08-2005 | NONE | |
| WO 03026647 A | 03-04-2003 | BR 0212044 A | 17-08-2004 |
| | | CA 2456606 A1 | 03-04-2003 |
| | | CN 1555262 A | 15-12-2004 |
| | | HR 20040085 A2 | 31-08-2004 |
| | | HU 0401567 A2 | 28-01-2005 |
| | | JP 2005503427 T | 03-02-2005 |
| | | MX PA04002583 A | 18-06-2004 |
| | | NO 20041176 A | 21-06-2004 |
| | | US 2004248944 A1 | 09-12-2004 |
| | | ZA 200402099 A | 26-04-2005 |
| WO 2005074920 A | 18-08-2005 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HOLLISTER.** *Pharm. Rev.,* 1986, vol. 38, 1-20
- **RENY ; SINGHA.** *Prog. Drug. Res.,* 1991, vol. 36, 71-114
- **CONSROE ; SANDYK.** Marijuana/Cannabinoids, Neurobiology and Neurophysiology. CRC Press, 1992
- *Synthetic communications,* 1996, vol. 26 (11), 2229-33
- *Synlett,* 2001, 147-149
- **KROGSGAARD-LARSEN et al.** Textbook of Drugdesign and Discovery. Taylor & Francis, April 2002
- **RUTH A. ROSS ; HEATHER C. BROCKIE et al.** Agonist-inverse agonist characterization at CB1 and CB2 cannabinoid receptors of L-759633, L759656 and AM630. *British Journal of Pharmacology,* 1999, vol. 126, 665-672
- **A. C. HOWLETT et al.** International Union of Pharmacology XXVII. Classification of Cannabinoid Receptors. *Pharmacol Rev,* 2002, vol. 54, 161-202
- **DAVID R. COMPTON et al.** In-vivo Characterization of a Specific Cannabinoid Receptor Antagonist (SR141716A) :Inhibition of Tetrahydrocannbinol- induced Responses and Apparent Agonist Activity. *J. Pharmacol. Exp. Ther.,* 1996, vol. 277 (2), 586-594
- **WOOLFE D. et al.** The evaluation of analgesic action of pethidine hydrochloride (Demerol. *J. Pharmacol. Exp. Ther.,* 1944, vol. 80, 300-307
- **DESMET L. K. C. et al.** Anticonvulsive properties of Cinarizine and Flunarizine in Rats and Mice. *Arzneim. -Forsch. (Frug Res,* 1975, vol. 25, 9
- **DAVID R. COMPTON et al.** In-vivo Characterization of a Specific Cannabinoid Receptor Antagonist (SR141716A) Inhibition of Tetrahydrocannbinol- induced Responses and Apparent Agonist Activity. *J. Pharmacol Exp Ther.,* 1996, vol. 277 (2), 586-594
- **ALPERMANN H. G. et al.** Pharmacological effets of Hoe 249: A new potential antidepressant. *Drugs Dev. Res.,* 1992, vol. 25, 267-282
- **G. COLOMBO et al.** Appetite Suppression and Weight Loss after the Cannabinoid Antagonist SR 141716. *Life Sciences,* 1998, vol. 63 (8), 113-117
- **E.T. TZAVARA et al.** The CB1 receptor antagonist SR141716A selectively increases monoaminergic neurotransmission in the medial prefrontal cortex: implications for therapeutic actions. *Br. J. Pharmacol.,* 2003, vol. 138 (4), 544-553